# EUROPEAN PATENT SPECIFICATION

(11) **EP 3 077 027 B1**
(45) Date of publication and mention of the grant of the patent: **16.10.2019**
(21) Application number: 14867159.7
(22) Date of filing: 02.12.2014
(51) Int. Cl.: A61M 5/32

(54) **A SAFETY CLIP**
SICHERHEITSKLAMMER
PINCE DE SÉCURITÉ

(30) Priority: 05.12.2013 AU 2013904740
(43) Date of publication of application: 12.10.2016
(73) Proprietor: Metier Medical Limited, Bundall, QLD 4217 (AU)
(72) Inventor: MINIX, Larry, Pontevedra Beach, FL (US)
(74) Representative: ip21 Ltd
(86) International application number: PCT/AU2014/001090
(87) International publication number: WO 2015/081370

(56) References cited:
- EP-A1- 2 676 693
- WO-A1-2013/029529
- US-A- 5 405 332
- US-A- 5 662 617
- US-A1- 2002 193 744
- US-A1- 2005 065 481
- US-A1- 2006 270 979

## Description

### TECHNICAL FIELD

The field of the present invention lies in medical devices and particularly, safety devices to minimise the risk of needlestick injuries.

### BACKGROUND ART

Medical staff need to routinely administer pharmaceutical products or withdraw bodily fluids by way of a hypodermic needle inserted into the body of the patient. These staff must dispose of the used needles in a safe and effective manner. Whilst containers have been in use to accept used needles and syringes, the containers do not address the risks to the medical staff after the injection or withdrawal of fluids and before the needle and syringe are inserted into the container.

In order to address this problem, certain sheaths and/or safety clips have been devised to be attached to the needle and/or syringe. However these clips have suffered problems with reliability and have not been taken up by the medical sector. It is an object of the current invention to substantially ameliorate some of the deficiencies of the prior art.

WO2013/029529 A1 illustrates one example of a prior art needle protection device. The needle protection device disclosed therein includes a connecting portion configured to be engaged with a needle hub. A protection arm, to cover the needle, is rotatably engaged with the connecting portion via a hinge member. The connecting portion has a through hole with paws adapted to receive an anchor head of a holding member on the protection arm so as to retain same in an unretreatable manner. This provides that, when the needle protection device is moved into the fully closed or full protection position, it is locked in position permanently, preventing the used safety needle from being used again. However, the needle protection device has issues in that during use of the needle i.e. when the protection arm is disengaged, the protection arm can interfere with the line of sight of the medical practitioner so they cannot clearly see the needle tip. This can lead to the medical practitioner not accurately positioning the needle tip and inadvertently coming into contact with it.

### DISCLOSURE OF INVENTION

According to the present invention, there is provided a safety clip for a needle, the safety clip comprising:
an attachment collar having a central aperture through which can be secured either a hub of a syringe with the needle or a collar at the base of the needle,
an elongate sheath having a longitudinal channel along at least one side thereof for receiving the needle,
a hinge operably connected between the attachment collar and the elongate sheath,
wherein the attachment collar further comprises one or more slots containing retaining members, the slots being located at a remote end of the attachment collar, which remote end is furthest from an end of the attachment collar to which the hinge is connected,
and wherein the elongate sheath further comprises one or more mushroom shaped projections which extend from a remote end of the elongate sheath in a direction parallel to the longitudinal channel, and are insertable into the slots and engage with the retaining members,
such that the elongate sheath of the safety clip, when the safety clip is secured by its attachment collar to a hub of a syringe with the needle or to a collar at the base of the needle, can be pivoted about the hinge between a first position in which the needle is exposed and able to be used, and a second position in which at least a tip of the needle is sheathed in the longitudinal channel so that a user cannot be pricked by the needle,
and wherein the retaining members are shaped to prevent the mushroom shaped projections being removed from their engagement with the retaining members, thereby providing a permanent and secure locking between the slots and the mushroom shaped projections which prevents the elongate sheath being pivoted back to the first position when placed in the second position, wherein the safety clip further comprises a temporary locking mechanism coupled between the elongate sheath and the attachment collar, the temporary locking mechanism being configured to selectively retain the safety clip in the first position so that the needle is exposed and available for use, wherein the temporary locking mechanism includes a first locking member coupled to the elongate sheath and a second locking member coupled to the attachment collar, the first and second locking members including respective distal ends configured to interface and lock with each other when the elongate sheath and attachment collar are fully extended in the first position.

Preferably, the or each slot contains three retaining members.

In a preferred form, there are two slots and there are two mushroom shaped projections.

In a further preferred form, the or each mushroom shaped projection has a recess and a ramped portion which has a thickest point at which it is slightly
wider than the width of an opening in the or each slot, the opening being defined between the retaining members, such that the ramped portion must be pushed into the slot as far as the recess so that the mushroom shaped projection is prevented from being removed from its engagement with the retaining members.

The elongate sheath preferably comprises retaining tabs for further retaining the needle in the second position.

It is also preferred that the retaining tabs project from at least one side of the longitudinal channel, the retaining tabs being temporarily displaced to allow the needle to be received within the longitudinal channel when the elongate sheath is being pivoted to the second position.

The retaining tabs preferably project at an angle such as to prevent the needle being dislodged from the elongate sheath.

Still more preferably, the elongate sheath, when used to sheath the needle, encloses the needle completely from one side, including the tip.

According to a second aspect of the invention, there is provided a safety needle assembly comprising the safety clip defined above connected to a collar at the base of a needle in a unitary construction.

According to a third aspect of the invention, there is provided a safety syringe assembly comprising the safety clip defined above connected to a hub of a syringe with a needle in a unitary construction.

### BRIEF DESCRIPTION OF THE DRAWINGS

Reference is now made to the drawings which depict
Fig. 1 is a perspective view of an embodiment of the safety clip not in accordance with the invention, the safety clip being shown with the attachment collar fully pivoted open about the hinge from the elongate sheath;
Fig. 2 is a longitudinal cross section of the safety clip as shown in Fig. 1 but upside down;

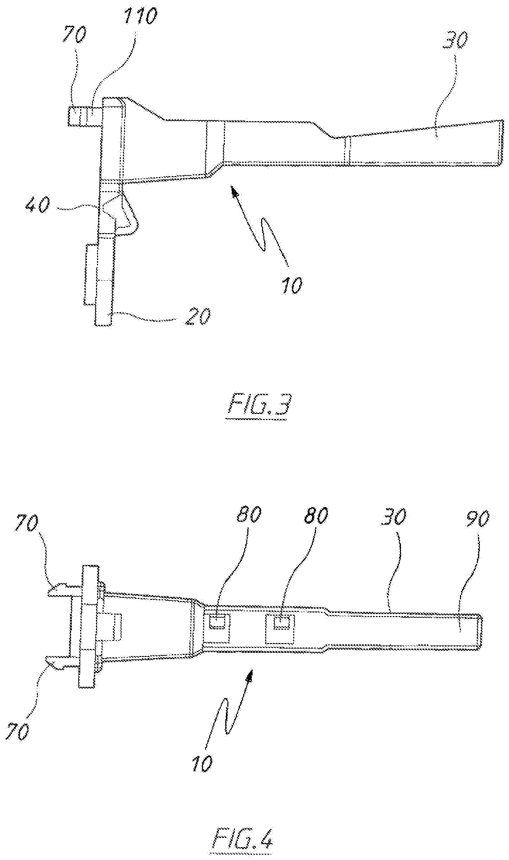

Fig. 3 is a longitudinal side view of the safety clip as shown in Fig. 1
Fig. 4 is a longitudinal bottom view of the safety clip as shown in Fig. 1

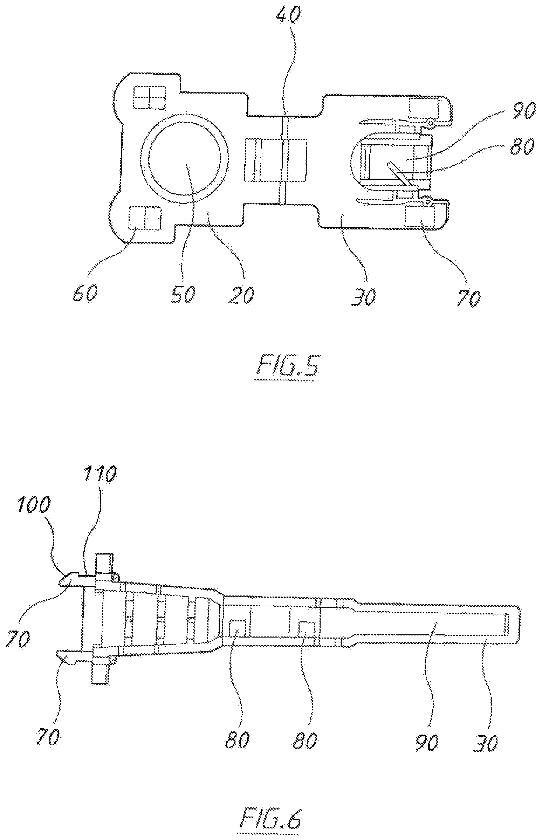

Fig. 5 is an end view from a pivoting end, at which the hinge is located, of the safety clip as shown in Fig. 1, but sideways;
Fig. 6 is a longitudinal top view, showing the longitudinal channel, of the safety clip as shown in Fig. 1;
Fig. 7 is an end view from a non pivoting end of the safety clip as shown in Fig. 1, but sideways;
Fig 8 is a longitudinal top view, showing only a portion of the longitudinal channel, of a second embodiment of the safety clip of the present invention, the safety clip being shown with the attachment collar only about half pivoted open about the hinge from the elongate sheath;
Fig. 9 is a perspective view of the safety clip as shown in Fig. 8;
Fig 10 is a different perspective view of the safety clip as shown in Fig. 8, and
Fig. 11 is a perspective view of the safety clip as shown in Fig. 8, the safety clip being shown with the attachment collar fully pivoted open about the hinge from the elongate sheath, and with the attachment collar securing the safety clip to a hub of a syringe so that the elongate sheath is in the first position in which a needle of the syringe js exposed and able to be used.

### BEST MODES FOR CARRYING OUT THE INVENTION

Prior art safety sheaths, clips and covers have suffered from several problems as set out in the background to the invention.

The present invention is adapted to be used with either a syringe which may have a detachable needle inserted therafter, or a needle alone, whereupon the assembly of needle and safety clip can be inserted onto the hub of a syringe. In alternate embodiments, the safety clip may form a portion of a combined syringe/clip/needle assembly or may be separate to a combined syringe/needle.

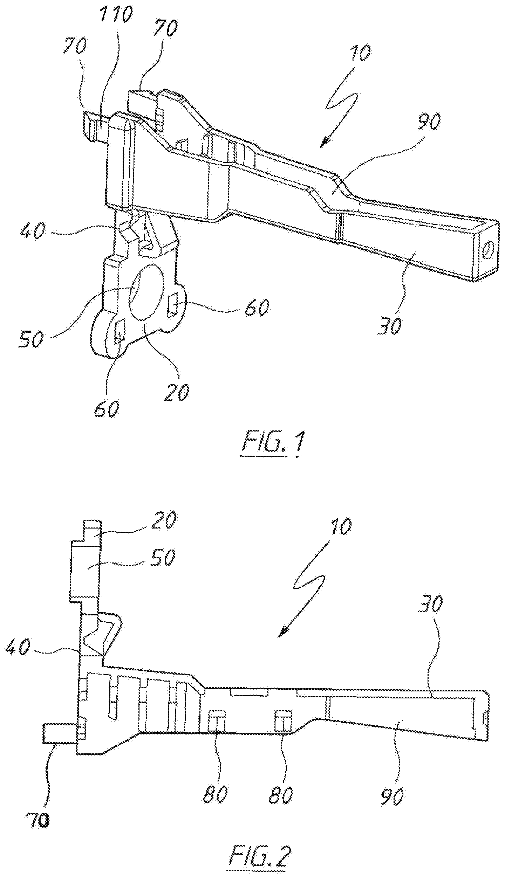

Referring to Fig 1 there is depicted a safety clip 10, not in accordance with the invention which is comprised of an attachment collar 20, elongate sheath 30 and hinge 40. The attachment collar 20 has a central aperture 50 through which either a hub of a syringe or the collar of the needle base is inserted and maintained in place through friction or alliteratively via locking means. Attachment collar 20 is further comprised oi 15 slots 60 which engage with tabs 70 of the elongate sheath 30. The elongate sheath 30 also has a longitudinal channel 90 for receiving a needle.

In use, the safety clip 10 can be configured to be in one of two positions about the hinge 40. In a first position, the needle is exposed and the slots 60 are not engaged with tabs 70. The safety clip 10 can be configured to be in a 20 second position by pivoting the elongate sheath 30 at the hinge 40 such that the channel 90 encases the needle and, with the application of pressure, causes the tabs 70 to be received or inserted into slots 60. Tabs 70 have a ramped portion 100 which, at its thickest point, is slightly wider than the width of the slot 60, such that the ramped portion 100 needs to be pushed into the slot where it is prevented from being removed from the slot 60 due to a recess 110.

The elongate sheath 30 also has retaining tabs 80 which retain the encased needle within the sheath. Retaining tabs 80 are formed as projections from one or either side of the channel 90 which are temporarily displaced when the needle is initially inserted into the channel 90, and thereafter are angled such that they prevent the needle from being dislodged out of the elongate sheath 30.

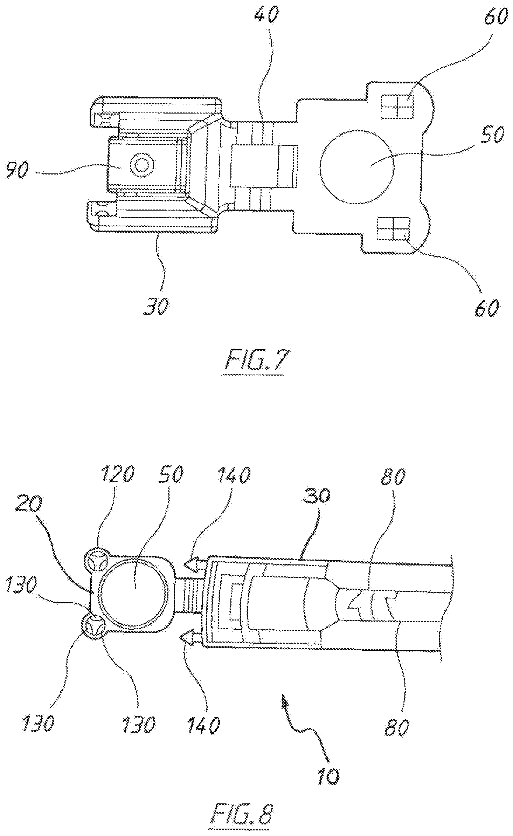

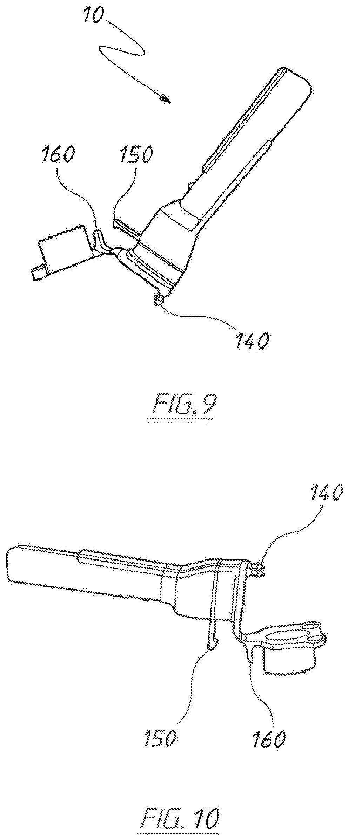

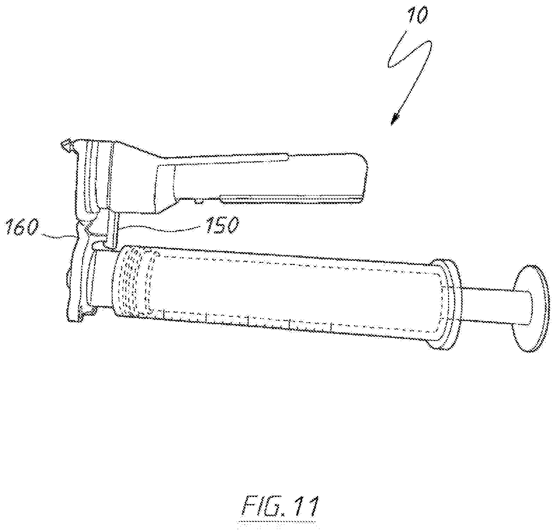

In an embodiment of the invention, as shown in Fig. 8, the attachment collar 20 has slots or apertures 120 containing retaining members 130 which engage with mushroom shaped projections 140 which are located on the elongate sheath 30. The retaining members 130 are shaped to allow the mushroom shaped projections 140 to enter the apertures 120 and the shape of the mushroom shaped projections 140 prevent their removal from the apertures 120. This embodiment, as depicted in Figs. 8 to 11, also depicts a second locking mechanism comprised of locking members 150 and 160 which operate to retain the safety clip 10 in the first or open position, where (as depicted in Fig. 11) the needle is not encased and the clip is retained against, in use, the barrel of the syringe, so as to prevent the clip from obscuring the view of the user of the syringe when giving an injection or removing bodily fluids. This second locking mechanism does not form a permanent lock, but rather a temporary fixing that can be released with minimum force.

The safety clip 10 can be provided on its own so that it can be used with any standard syringes that have luer locks or luer slip locks. Alternatively, the safety clip can be incorporated into a kit comprising a safety clip 10 and a syringe. In such case, the syringe and safety clip 10 can be made as a single component in a unitary construction. Alternatively, the safety clip 10 can be provided in a kit with a needle only. In such cases, the needle and safety clip 10 can be made as a single component in a unitary construction, and attached to any standard luer lock or luer slip Jock syringe.

## Claims

1. A safety clip for a needle, the safety clip comprising:
an attachment collar (20) having a central aperture (50) through which can be secured either a hub of a syringe with the needle or a collar at the base of the needle, an elongate sheath (30) having a longitudinal channel (90) along at least one side thereof for receiving the needle, a hinge operably connected between the attachment collar (20) and the elongate sheath (30), wherein the attachment collar (20) further comprises one or more slots (120) containing retaining members (130), and wherein the elongate sheath further comprises one or more mushroom shaped projections (140) which extend from a remote end of the elongate sheath (30) in a direction parallel to the longitudinal channel, and are insertable into the slots and engage with the retaining members (130), such that the elongate sheath of the safety clip, when the safety clip is secured by its attachment collar (20) to a hub of a syringe with the needle or to a collar at the base of the needle, can be pivoted about the hinge between a first position in which the needle is exposed and able to be used, and a second position in which at least a tip of the needle is sheathed in the longitudinal channel so that a user cannot be pricked by the needle, and wherein the retaining members (130) are shaped to prevent the mushroom shaped projections (140) being removed from their engagement with the retaining members, thereby providing a permanent and secure locking between
the slots (120) and the mushroom shaped projections (140) which prevents the elongate sheath (30) being pivoted back to the first position when placed in the second position, **characterized in that** the slots (120) are located at a remote end of the attachment collar (20), which remote end is furthest from an end of the attachment collar to which the hinge is connected, and **in that** the safety clip further comprises a temporary locking mechanism coupled between the elongate sheath and the attachment collar, the temporary locking mechanism being configured to selectively retain the safety clip in the first position so that the needle is exposed and available for use, wherein the temporary locking mechanism includes a first locking member (150) coupled to the elongate sheath and a second locking member (160) coupled to the attachment collar, the first and second locking members including respective distal ends configured to interface and lock with each other when the elongate sheath and attachment collar are fully extended in the first position.

2. The safety clip of claim 1 wherein the or each slot (120) contains three retaining members (130).

3. The safety clip of claim 1 wherein there are two slots (120) and there are two mushroom shaped projections (140).

4. The safety clip of claim I wherein the or each mushroom shaped projection (140) has a recess and a ramped portion which has a thickest point at which it is slightly wider than the width of an opening in the or each slot (120), the opening being defined between the retaining members (130), such that the ramped portion must be pushed into the slot as far as the recess so that the mushroom shaped projection (140) is prevented from being removed from its engagement with the retaining members (130).

5. The safety clip of claim 1 wherein the elongate sheath (30) comprises retaining tabs (80) for further retaining the needle in the second position.

6. The safety clip of claim 5 wherein the retaining tabs (80) project from at least one side of the longitudinal channel (90), the retaining tabs (80) being temporarily displaced to allow the needle to be received within the longitudinal channel (90) when the elongate sheath is being pivoted to the second position.

7. The safety clip of claim 6 wherein the retaining tabs (80) project at an angle such as to prevent the needle being dislodged from the elongate sheath (30).

8. The safety clip of claim 1 wherein the elongate sheath (30), when in the second position, encloses the needle completely from one side.

9. A safety needle assembly comprising the safety clip of claim 1 connected to a collar at the base of a needle in a unitary construction.

10. A safety syringe assembly comprising the safety clip of claim 1 connected to a hub of a syringe with a needle in a unitary construction.

## Patentansprüche

1. Sicherheitsclip für eine Nadel, wobei der Sicherheitsclip umfasst:
einen Befestigungskragen (20) mit einer zentralen Öffnung (50), durch die entweder eine Nabe einer Spritze mit der Nadel oder ein Kragen an der Basis der Nadel befestigt werden kann, eine längliche Hülle (30) mit einem Längskanal (90) entlang mindestens einer Seite derselben zur Aufnahme der Nadel, ein Scharnier, das funktionsfähig zwischen dem Befestigungskragen (20) und der länglichen Hülle (30) verbunden ist, wobei der Befestigungskragen (20) ferner einen oder mehrere Schlitze (120) umfasst, die Rückhalteelemente (130) enthalten, und wobei die längliche Hülle ferner einen oder mehrere pilzförmige Vorsprünge (140) umfasst, die sich von einem entfernten Ende der länglichen Hülle (30) in einer Richtung parallel zum Längskanal erstrecken, und sind in die Schlitze einsetzbar und greifen in die Rückhalteelemente (130) ein, so dass die längliche Hülle des Sicherheitsclips, wenn der Sicherheitsclip mit ihrem Befestigungskragen (20) an einer Nabe einer Spritze mit der Nadel oder an einem Kragen an der Basis der Nadel befestigt ist, um das Scharnier zwischen einer ersten Position, in der die Nadel freiliegt und verwendet werden kann, und einer zweiten Position, in der mindestens eine Spitze der Nadel in den Längskanal eingehüllt ist, schwenkbar ist, so dass ein Benutzer nicht von der Nadel gestochen werden kann, und wobei die Rückhalteelemente (130) so geformt sind, dass verhindert wird, dass die pilzförmigen Vorsprünge (140) aus ihrem Eingriff mit den Rückhalteelementen entfernt werden, wodurch eine dauerhafte und sichere Verriegelung zwischen den Schlitzen (120) und den pilzförmigen Vorsprüngen (140) bereitgestellt wird, die verhindert, dass die längliche Hülle (30) in der zweiten Position wieder in die erste Position geschwenkt wird, wenn sie in die zweite Position gebracht wird, **dadurch gekennzeichnet, dass** die Schlitze (120) an einem entfernten Ende des Befestigungskragens (20) angeordnet sind, wobei das entfernte Ende am weitesten von einem Ende des Befestigungskragens entfernt ist, mit dem das Scharnier verbunden ist, und dass der Sicherheitsclip ferner einen temporären Verriegelungsmechanismus umfasst, der zwischen der länglichen Hülle und dem Befestigungskragen gekoppelt ist, wobei der temporäre Verriegelungsmechanismus konfiguriert ist, um den Sicherheitsclip selektiv in der ersten Position zu halten, so dass die Nadel freiliegt und für den Gebrauch zur Verfügung steht, worin der temporäre Verriegelungsmechanismus ein erstes Verriegelungselement beinhaltet, das mit der länglichen Hülle gekoppelt ist, und ein zweites Verriegelungselement, das mit dem Befestigungskragen gekoppelt ist, worin das erste und zweite Verriegelungselement entsprechende distale Enden beinhalten, die konfiguriert sind, um miteinander zu kommunizieren und zu verriegeln, wenn die längliche Hülle und der Befestigungskragen in der ersten Position vollständig ausgefahren sind.

2. Sicherheitsclip nach Anspruch 1, worin der oder jeder Schlitz (120) drei Halteelemente (130) enthält.

3. Sicherheitsclip nach Anspruch 1, worin zwei Schlitze (120) und zwei pilzförmige Vorsprünge (140) vorhanden sind.

4. Sicherheitsclip nach Anspruch 1, worin der oder jeder pilzförmige Vorsprung (140) eine Aussparung und einen rampenförmigen Abschnitt aufweist, der einen dicksten Punkt aufweist, an dem er etwas breiter ist als die Breite einer Öffnung in dem oder jedem Schlitz (120), wobei die Öffnung zwischen den Halteelementen (130) definiert ist, so dass der rampenförmige Abschnitt in den Schlitz bis zur Aussparung geschoben werden muss, so dass verhindert wird, dass der pilzförmige Vorsprung (140) aus seinem Eingriff mit den Halteelementen (130) entfernt wird.

5. Sicherheitsclip nach Anspruch 1, worin die längliche Hülle (30) Haltelaschen (80) zum weiteren Halten der Nadel in der zweiten Position umfasst.

6. Sicherheitsclip nach Anspruch 5, worin die Haltelaschen (80) von mindestens einer Seite des Längskanals (90) vorstehen, wobei die Haltelaschen (80) vorübergehend verschoben sind, um zu ermöglichen, dass die Nadel innerhalb des Längskanals (90) aufgenommen wird, wenn die längliche Hülle in die zweite Position geschwenkt wird.

7. Sicherheitsclip nach Anspruch 6, worin die Haltelaschen (80) in einem solchen Winkel vorstehen, dass verhindert wird, dass die Nadel aus der länglichen Hülle (30) gelöst wird.

8. Sicherheitsclip nach Anspruch 1, worin die längliche Hülle (30), wenn sie sich in der zweiten Position befindet, die Nadel von einer Seite vollständig umschließt.

9. Sicherheitsnadelanordnung, umfassend den Sicherheitsclip nach Anspruch 1, der mit einem Kragen an der Basis einer Nadel in einer einheitlichen Konstruktion verbunden ist.

10. Sicherheitsspritzenanordnung, umfassend den Sicherheitsclip nach Anspruch 1, die mit einer Nabe einer Spritze mit einer Nadel in einer einheitlichen Konstruktion verbunden ist.

## Revendications

1. Clip de sécurité pour aiguille, le clip de sécurité comprenant :
un collier de fixation (20) ayant une ouverture centrale (50) à travers laquelle peut être fixé soit un moyeu d'une seringue avec l'aiguille, soit un collier à la base de l'aiguille, une gaine allongée (30) ayant un canal longitudinal (90) sur au moins un côté de celle-ci pour recevoir l'aiguille, une articulation reliée de manière opérationnelle entre le collier de fixation (20) et la gaine allongée (30), dans laquelle le collier de fixation (20) comprend en outre une ou plusieurs fentes (120) contenant des éléments de retenue (130) et dans laquelle la gaine allongée comprend en outre une ou plusieurs saillies (140) en forme de champignon qui s'étendent depuis une extrémité distante de la gaine allongée (30) dans une direction parallèle au canal longitudinal, et sont insérables dans les fentes et s'engagent dans les éléments de retenue (130), de telle sorte que la gaine allongée du clip de sécurité, lorsque le clip de sécurité est fixée par son collier de fixation (20) à un moyeu d'une seringue avec l'aiguille ou à un collier à la base de l'aiguille, peut être pivotée autour de l'articulation entre une première position dans laquelle l'aiguille est exposée et peut être utilisée et une seconde position dans laquelle au moins une extrémité de l'aiguille est recouverte dans le canal longitudinal afin que l'utilisateur ne puisse être piqué par l'aiguille, et dans lequel les éléments de retenue (130) sont formés de manière à empêcher que les saillies en forme de champignon (140) ne soient retirées de leur engagement avec les éléments de retenue, assurant ainsi un verrouillage permanent et sûr entre les fentes (120) et les saillies en forme de champignon (140) qui empêche le pivotement de la gaine allongée (30) dans la première position quand elle est placée en deuxième position, **caractérisé en ce que** les fentes (120) sont situées à une extrémité distante du collier de fixation (20), laquelle extrémité distante est la plus éloignée d'une extrémité du collier de fixation auquel est reliée l'articulation, et **en ce que** le clip de sécurité comprend en outre un mécanisme de verrouillage temporaire couplé entre la gaine allongée et le collier de fixation, le mécanisme de verrouillage temporaire étant configuré pour retenir sélectivement le clip de sécurité dans la première position afin que l'aiguille soit exposée et disponible pour utilisation, dans lequel le mécanisme de verrouillage temporaire comprend un premier élément de verrouillage couplé à la gaine allongée et un deuxième élément de verrouillage couplé au collier de fixation, les premier et deuxième éléments de verrouillage comprenant les extrémités distales respectives configurées pour s'interfacer et se verrouiller les uns avec les autres quand la gaine allongée et le collier de fixation sont entièrement déployés dans la première position.

2. Clip de sécurité selon la revendication 1, dans lequel la ou chaque fente (120) contient trois éléments de retenue (130).

3. Clip de sécurité selon la revendication 1, dans lequel il y a deux fentes (120) et deux saillies en forme de champignon (140).

4. Clip de sécurité selon la revendication 1, dans lequel la ou chaque saillie en forme de champignon (140) présente un évidement et une partie en rampe qui présente un point le plus épais au niveau duquel elle est légèrement plus large que la largeur d'une ouverture dans la ou chaque fente (120), l'ouverture étant définie entre les éléments de retenue (130), de sorte que la partie en rampe doit être enfoncée dans la fente jusqu'à l'évidement afin que la saillie en forme de champignon (140) ne soit pas enlevée de son engagement avec les éléments de retenue (130).

5. Clip de sécurité selon la revendication 1, dans lequel la gaine allongée (30) comprend des pattes de retenue (80) pour retenir davantage l'aiguille dans la seconde position.

6. Clip de sécurité selon la revendication 5, dans lequel les pattes de retenue (80) font saillie d'au moins un côté du canal longitudinal (90), les pattes de retenue (80) étant temporairement déplacées pour permettre à l'aiguille d'être reçue dans le canal longitudinal (90) lorsque la gaine allongée est pivotée dans la deuxième position.

7. Clip de sécurité selon la revendication 6, dans lequel les pattes de retenue (80) font saillie selon un angle tel qu'elles empêchent que l'aiguille ne soit délogée de la gaine allongée (30).

8. Clip de sécurité selon la revendication 1, dans lequel la gaine allongée (30), lorsqu'elle est dans la deuxième position, entoure complètement l'aiguille d'un côté.

9. Ensemble d'aiguille de sécurité comprenant le clip de sécurité selon la revendication 1 relié à un collier à la base d'une aiguille dans une construction unitaire.

10. Ensemble de seringue de sécurité comprenant le clip de sécurité selon la revendication 1 relié à un moyeu d'une seringue avec une aiguille dans une construction unitaire.
